# EUROPEAN PATENT APPLICATION

(11) **EP 1 997 461 A2**
(43) Date of publication of application: **03.12.2008**
(21) Application number: 08425371.5
(22) Date of filing: 23.05.2008
(51) Int. Cl.: A61F 9/007

(54) **Scalpel for ophthalmic surgery with integrated temperature measurement, and process for realizing the same**

(30) Priority: 29.05.2007 IT RM20070293
(71) Applicant: Optikon 2000 S.p.A., 00138 Roma (IT)
(72) Inventor: De Cesare, Giampiero, 00184 Roma (IT); Angelini, Giovan Battista, 00138 Roma (IT)
(74) Representative: Iannone, Carlo Luigi

(57) **Abstract**

The invention relates to a scalpel (1) for ophthalmic surgery with integrated temperature measurement characterized in that it provides a temperature sensor (2) comprised of biocompatible material, provided on the end of the needle (1) and connected with an electronic circuit for processing the signal.

The invention further concerns a process for manufacturing the above described scalpel (1) for ophthalmic surgery.

## Description

The present invention concerns a scalpel for ophthalmic surgery with integrated temperature measurement.

More specifically, the invention concerns a system able detecting local temperature of the eye during the surgical intervention.

The invention further relates to a reliable and economic process for realizing said scalpel that can be carried out at low temperature.

As it is well known, measurement of temperature is a very interesting parameter for the cataract surgery.

Phaco-emulsification intervention widely diffused in the last decade for removing cataract.

Substantially, a titanium needle (with a diameter of about 1 mm), connected with a suction pump, is axially oscillated at a frequency of about 40 KHz. Needle is then brought in contact with crystalline with cataract to be removed which is crashed (emulsified) into small particles, and sucked through the same tip. Sucked material is replaced by balanced saline solution input within the eye, usually by a silicone sleeve, which is coaxial with respect to the sucking tip.

If during the intervention torsions are exerted on the tip or if incision through which the sleeve-tip assembly enters within the patient eye is not sufficiently wide, it can occur that oscillating tip comes into contact with irrigation sleeve and that irrigating solution is ejected from the contact zone. If at the same time sucking flow is reduced or blocked since tip clogged by cataract fragments to be removed, heat is developed by friction between tip and sleeve, quickly raising local temperature, until producing cornea deformations and in some cases even burns.

Said traumas slow down healing process after the intervention and can cause permanent deformations (serious astigmatism).

Many solutions have been suggested during the years in order to try to reduce the incidence of the problem caused by indirect solutions, such as creation of micro-interruptions when emitting ultrasounds in order to reduce delivered power (U.S. patent n° 6,780,165) or trying calculating temperature reached at the incision level starting from the energy balance between power input within the eye and that removed by sucked fluids (U.S. patent n° 6,999,212).

Possibility of measuring temperature of the contact point would allow immediately blocking oscillation of the tip in case of overheating, thus preventing any damage.

Other solutions suggested provide two systems with temperature sensors provided within the silicone sleeve, surrounding the tip (U.S. patent n° 6,193,683).

This kind of approach, even being an improvement with respect to the previous solutions, has in any case some drawbacks, since:
- position of the sleeve must be adjusted with respect to the tip, so that the sleeve must be rotated and advanced; this brings to serious difficulties for realizing connections between sensors and control electronics;
- some surgical techniques developed in the recent years provide the use of the phaco-emulsification tip without the sleeve and with irrigation by a second incision (bimanual techniques).

In view of the above, the Applicant has realised a solution permitting facing and solving the above mentioned problems, placing one or more sensors directly on the phaco-emulsification tip, at the height of the surgical incision.

The solution according to the invention starts from the consideration that temperature sensors are realised since many time with different techniques: thermocouple, thermo-resistance, crystalline silicium diode. Their use is partially determined by the particular application and by the temperature range to be measured.

These and other results are obtained according to the invention by a system basically comprised of a temperature sensor, made up of biocompatible material, micro-manufactured on the end of a titanium needle used during the intervention, and connected by thin film techniques, with an electronic circuit for processing the signal.

It is therefore specific object of the present invention a scalpel for ophthalmic surgery with integrated temperature measurement characterized in that it provides a temperature sensor comprised of biocompatible material, provided on the end of the needle and connected with an electronic circuit for processing the signal.

Preferably, according to the invention, said temperature sensor is micro-manufactured on the end of the scalpel needle.

Furthermore, according to the invention, said temperature sensor is connected with the electronic circuit by thin film techniques.

In a preferred embodiment of the scalpel according to the invention, said sensor is comprised by an overlapped layer amorphous silicium joint, with a two electric contacts structure, one of which is comprised of the needle, preferably a titanium needle, and the other one is comprised of a metallic film, thermally evaporated under vacuum, and shaped in order to brig sensor contact at the basis of the scalpel.

Furthermore, according to the invention, extraction of the sensor signal toward electronic reading circuit occurs connecting a shielded cable on the metallic track by sliding contacts, or using conductive glues, or by ultrasound micro-welding, usually employed in the microelectronic field.

Always according to the invention, close to the sensor, an insulating material film is interposed between the metallic track and the needle, with an opening in correspondence of the sensor.

The invention further relates to a process for manufacturing the above described scalpel, providing all or only some of the following steps:
a. mechanical and/or chemical lapping by etching within acid solution of the needle surface;
b. deposition of the hydrogenated amorphous silicium structure, by plasma assisted vapour phase chemical deposition (PECVD)
c. sputtering deposition of a metallic thin film (< 1 micrometer), through a steel mask with a hole in correspondence of the needle end, having a diameter equal to the sensor dimensions;
d. definition of the temperature sensor area, by photolithographic process and selective attack to the amorphous silicium (RIE);
e. deposition of an insulating layer (e.g. silicium nitride, polymer film, ecc.);
f. opening of the hole for electric connection with sensor, by plasma attack (RIE) through the same steel shield employed for defining the sensor;
g. deposition, by thermal evaporation under vacuum, of a metallic track from sensor to scalpel base;
h. deposition of a protection insulating layer over the whole structure, up to the needle base;
i. realisation by sliding contacts of the electric contact between track and electronic circuit for reading the sensor, or by conductive glues, or by ultrasound micro-welding, usually employed in the microelectronics field.

The present invention will be now described, for illustrative but not limitative purposes, according to its preferred embodiments, with particular reference to the figures of the enclosed drawings, wherein:
figure 1 schematically shows a scalpel for ophthalmic surgery according to the invention;
figure 2 schematically shows the process for realising scalpel of figure 1;
figure 3 shows the current -tension features of the device according to the invention;
figure 4 shows a graph relevant to the connection between sensor temperature of figure 3 and tension measure with a constant current of 10 nA; and
figure 5 shows a graph relevant to relation between needle temperature and current measured at a constant current of 0.2 V.

Observing now the figures of the enclosed drawings, it will be described a scalpel according to the invention, making also reference to a process for its realisation.

Application of a, ophthalmic scalpel has such specifications to make it very difficult the use of standard manufacturing techniques. In fact, titanium needle, during the intervention is axially oscillated at a 40 kHz frequency, with amplitude of about 0.1 mm, so that sensor to be realised must withstand to the mechanical stresses due to the acceleration during oscillation, without dislodging from its seat.

The solution suggested according to the present invention uses the manufacturing technology of amorphous silicium thin film, by which it is possible realising both active electronic devices (MOS transistors) and sensors for various applications (optic, mechanical chemical). Particularly, it is possible employing joint devices with p-type/intrinsic/n-type silicium multilayer structures, with few nanometres of thickness, directly on metallic substrates, with rectified current-tension features, and depending on the joint temperature.

Furthermore, amorphous silicium can be deposited by plasma ionic discharge (Plasma Enhanced Chemical Vapour Deposition: PECVD), i.e. a suitable deposition technology, permitting manufacturing of devices on curve surfaces.

Moreover, deposition temperature, about 200°C, is compatible with different kind of substrates, such as steel glass or plastic, obtaining a high adherence of film on different substrates and the structure e can be read by a low tension electronic device.

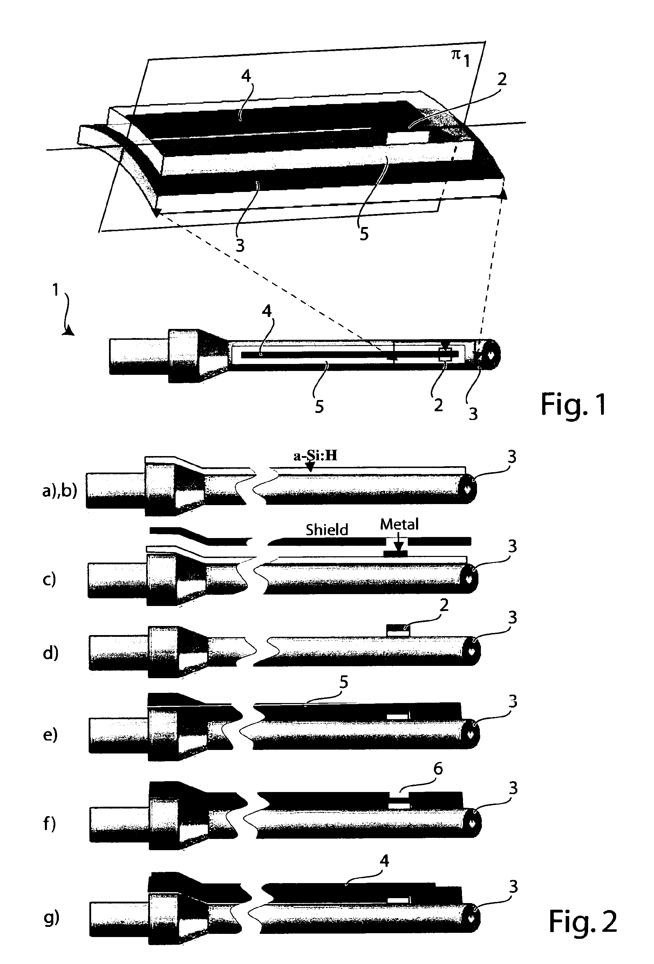

Making now specific reference to figure 1, it is shown a scalpel needle for ophthalmic surgery, generically indicated by reference number 1, wherein it is deposited temperature sensor 2, by standard thin film techniques, directly on the end part of the titanium needle used during the surgical intervention.

Sensor 2 evidenced in the enlarged particular of figure 1 is comprised of an amorphous silicium joint, with overlapped layers, in such a way to realise a two electric contacts structure, that, if suitably polarised, make the diode active as temperature sensor.

One of the two metallic contacts of sensors 2 is support titanium needle 1, while upper electrode is comprised of a metallic film thermally evaporated under vacuum, and shaped in such a way to bring sensor 2 contacts at the base of the scalpel 1.

Extraction of sensor 2 signal toward reading electronic circuit occurs connecting a shielded cable on the metallic track 4 by sliding contacts, or employing conductive glues, or by ultrasound micro-welding usually employed in the microelectronics.

As put into evidence in the enlarged particular of the structure close to sensor 2, between metallic track 4 and needle 1 it is necessary interposing an insulating material film 5, with an opening in correspondence of sensor 2.

In the following, making specific reference to figure 2, it will be described a process for manufacturing scalpel 1 according to the invention.

The process according to the invention provides all or only some of the following steps:
a. mechanical and/or chemical lapping by etching within acid solution of the needle 1 surface;
b. deposition of the hydrogenated amorphous silicium structure, by plasma assisted vapour phase chemical deposition (PECVD)
c. sputtering deposition of a metallic thin film (< 1 micrometer), through a steel mask with a hole in correspondence of the needle 1 end, having a diameter equal to the sensor dimensions;
d. definition of the temperature sensor area, by photolithographic process and selective attack to the amorphous silicium (RIE);
e. deposition of an insulating layer (e.g. silicium nitride, polymer film, ecc.);
f. opening of the hole for electric connection with sensor, by plasma attack (RIE) through the same steel shield employed for defining the sensor;
g. deposition, by thermal evaporation under vacuum, of a metallic track from sensor to scalpel base;
h. deposition of a protection insulating layer over the whole structure, up to the needle 1 base;
i. realisation by sliding contacts of the electric contact between track and electronic circuit for reading the sensor, or by conductive glues, or by ultrasound micro-welding, usually employed in the microelectronics field.

In a first prototype realised according to the invention, temperature sensor has been realised with a stacked joint with overlapped layers of amorphous silicium of the n-type/intrinsic/p-type kind, with 30/300/30 nanometres, respectively. Device was fabricated on the titanium needle, in a high vacuum system.

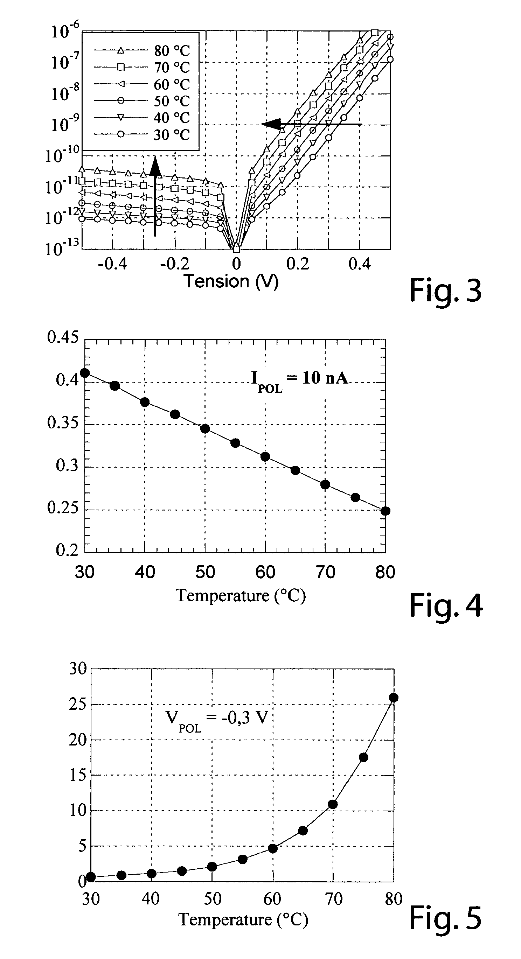

Figure 3 shows current - tension features (log |I| - V) of device, for temperatures between 30°C and 80°C, measured contacting with positive electrode the metallic film deposited on the p-type state of device and negative electrode directly on the needle.

Features strongly depend on temperature and particularly with an increase of temperature curves linearly translate on the left for positive tensions, and exponentially upward (increase of the opposite current of joint) for negative tensions.

By the inventive system it will be possible detecting needle temperature, suitably polarising diode through two electric contacts, one made up directly by the titanium needle and the other one by the metallic track, shaped in such a way to bring contact from sensor to the base of the same scalpel.

Particularly, directly polarising the sensor, by a constant current, tension value is linearly dependent on the joint temperature.

Graph of figure 4 shows relation between temperature of sensor shown in figure 3 and tension measured with a constant current of 10 nA. A sensibility of about 3.3 mV/°C is obtained on the basis of slope or line passing through the measured points.

Polarising sensor with a negative constant tension (positive electrode on the needle and negative electrode on metallic track) current passing through device is an exponential function of sensor temperature.

Finally, graph of figure 5 shows relation between needle temperature and current measured at a constant tension of 0.2 V.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Scalpel for ophthalmic surgery with integrated temperature measurement **characterized in that** it provides a temperature sensor comprised of biocompatible material, provided on the end of the needle and connected with an electronic circuit for processing the signal.

2. Scalpel for ophthalmic surgery with integrated temperature measurement according to claim 1, **characterized in that** said temperature sensor is micro-manufactured on the end of the scalpel needle.

3. Scalpel for ophthalmic surgery with integrated temperature measurement according to one of the preceding claims, **characterized in that** said temperature sensor is connected with the electronic circuit by thin film techniques.

4. Scalpel for ophthalmic surgery with integrated temperature measurement according to one of the preceding claims, **characterized in that** said sensor is comprised by an overlapped layer amorphous silicium joint, with a two electric contacts structure, one of which is comprised of the needle, preferably a titanium needle, and the other one is comprised of a metallic film, thermally evaporated under vacuum, and shaped in order to brig sensor contact at the basis of the scalpel.

5. Scalpel for ophthalmic surgery with integrated temperature measurement according to one of the preceding claims, **characterized in that** extraction of the sensor signal toward electronic reading circuit occurs connecting a shielded cable on the metallic track by sliding contacts, or using conductive glues, or by ultrasound micro-welding, usually employed in the microelectronic field.

6. Scalpel for ophthalmic surgery with integrated temperature measurement according to one of the preceding claims, **characterized in that**, close to the sensor, an insulating material film is interposed between the metallic track and the needle, with an opening in correspondence of the sensor.

7. Process for manufacturing the above described scalpel, providing all or only some of the following steps:
a. mechanical and/or chemical lapping by etching within acid solution of the needle surface;
b. deposition of the hydrogenated amorphous silicium structure, by plasma assisted vapour phase chemical deposition (PECVD)
c. sputtering deposition of a metallic thin film (< 1 micrometer), through a steel mask with a hole in correspondence of the needle end, having a diameter equal to the sensor dimensions;
d. definition of the temperature sensor area, by photolithographic process and selective attack to the amorphous silicium (RIE);
e. deposition of an insulating layer (e.g. silicium nitride, polymer film, ecc.);
f. opening of the hole for electric connection with sensor, by plasma attack (RIE) through the same steel shield employed for defining the sensor;
g. deposition, by thermal evaporation under vacuum, of a metallic track from sensor to scalpel base;
h. deposition of a protection insulating layer over the whole structure, up to the needle base;
i. realisation by sliding contacts of the electric contact between track and electronic circuit for reading the sensor, or by conductive glues, or by ultrasound micro-welding, usually employed in the microelectronics field.
